# EUROPEAN PATENT SPECIFICATION

(11) **EP 1 600 112 B1**
(45) Date of publication and mention of the grant of the patent: **31.01.2007**
(21) Application number: 05291088.2
(22) Date of filing: 20.05.2005
(51) Int. Cl.: A61B 17/70

(54) **Implants for a spinal osteosynthesis device, and assembly comprising these implants**
Implantate für eine Vorrichtung zur Wirbelsäulenosteosynthese und Anordnung mit diesen Implantaten
Implants pour un dispositif d'ostéosynthèse rachidienne et ensemble comprenant ces implants

(30) Priority: 26.05.2004 FR 0405691
(43) Date of publication of application: 30.11.2005
(73) Proprietor: Warsaw Orthopedic, Inc., Warsaw, IN 46581 (US)
(72) Inventor: Gournay, José, 77230 Dammartin en Goele (FR); Lemaitre, Philippe, 94140 Alfortville (FR); Banouskou, Ezzine, 93420 Villepinte (FR)
(74) Representative: Neyret, Daniel Jean Marie

(56) References cited:
- WO-A-02/38061
- US-A- 5 628 740
- US-A- 5 649 926
- US-A- 5 810 817
- US-A- 5 879 351

## Description

The invention concerns the field of implants intended to be included in spinal osteosynthesis devices for correcting deformations of the spine.

Devices of this kind often comprise a rod extending along a portion of the spine. In the sacral region, these rods are usually fixed to the sacrum of the patient by way of a plate which, for example, straddles vertebrae S1 and S2 and bears a protrusion on which a slotted connector is fitted and immobilized, which slotted connector is itself traversed by the rod and immobilizes the latter by a wedging action. An example of such a plate is described in document WO-A-02/38061, for example.

US-A-5,879,351 describes L-shaped connectors interconnecting a vertebral rod and a pedicle screw.

It is also known to provide a plate which is fixed no longer on vertebrae S1 and S2, but on one vertebra (for example S1) and on the iliac bone of the patient. For this purpose, the plate comprises, at one of its ends, a lateral extension provided with an orifice for the passage of a bone anchoring screw. This lateral extension is oriented in the direction of an iliac bone and is thus situated outside the plane of the plate, with which it normally forms an angle of the order of 50°. In this way, the iliac bone can be connected to the rod.

Different sizes of these sacral plates with iliac extension are available and are used in accordance with the morphology of the patient. However, the adaptation of the plate to the exact morphology of the patient is often only approximate.

US-A-5, 810, 817 shows an example of such an ilio-sacral plate.

The object of the invention is to provide surgeons with means which are used to connect the rod to an iliac bone and which can best adapt to the morphology of each patient while at the same time still being inexpensive to produce and easy to use.

To this end, the subject of the invention is an implant for a spinal osteosynthesis device, having the features recited in claim 1.

Said immobilizing means can be formed by an internally threaded hole and a threaded plug screwed into said internally threaded hole.

Said protrusion can have a multi-axial orientation inside a cone.

Said implant can comprise a dome whose inner surface is of conical shape; the surface of the lower part of the protrusion, intended to be held inside said dome and to come into contact with said inner surface of the dome, can be of spherical shape and be traversed by a slot delimited by an upper surface of V shape with the point directed towards the lower end of the protrusion, and said dome and said slot can be traversed by a pin.

The dome can have a spherical outer surface.

The invention also relates to an assembly formed by an implant for a spinal osteosynthesis device, a slotted connector for attachment to a rod, of the type comprising an orifice for fitting said connector on said protrusion, and a nut for immobilizing said connector on said implant and for clamping said rod in said connector, and said implant is of the above type in two parts.

The implant can be of the type comprising a dome with a spherical outer surface, and the surface delimiting said orifice of the connector can be of conical shape.

As will have been appreciated, the invention lies in connecting an iliac bone to the rod of the osteosynthesis device by means of a plate comprising an orifice in which a rod can be fitted and immobilized. This rod can be:
- either a rod forming a longitudinal continuation of a sacral plate, on a protrusion of which a connector is fitted which is itself traversed by the rod of the osteosynthesis device; this then is a sacral plate with a lateral extension of variable geometry, due to the fact that the position of the lateral extension on the rod of the plate can be chosen, both in terms of rotation and of translation, so as to best adapt to the exact anatomy of the patient;
- or the rod of the osteosynthesis device itself, which for this reason is attached directly to the iliac bone.

The invention will be better understood on reading the following description in which reference is made to the following attached figures, where:
- Figure 1 shows a perspective view of an example of an implant in two parts according to the invention;
- Figure 2 shows this same implant mounted on the sacrum and an iliac bone of a patient and included in a spinal osteosynthesis device;
- Figures 3 to 5 and Figure 7 show, in a perspective view (Fig. 3), a partial section (Fig. 4), and, for one of its components, a longitudinal section (Fig. 5), the sacral plate of the above implant in order to demonstrate the preferred multi-axial articulation of its protrusion;
- Figure 6 shows said sacral plate, a connector, and the nut which joins them;
- Figure 7 shows a partial cross section through the above sacral plate, connector and nut in the assembled state;
- Figure 8 shows a perspective view of a series of plates with lateral extensions, which can be used alone or in combination with a sacral plate to form an implant according to the invention;
- Figure 9 shows an implant not made according to the invention, formed exclusively of a lateral extension plate, mounted on an iliac bone of a patient and included in a spinal osteosynthesis device.

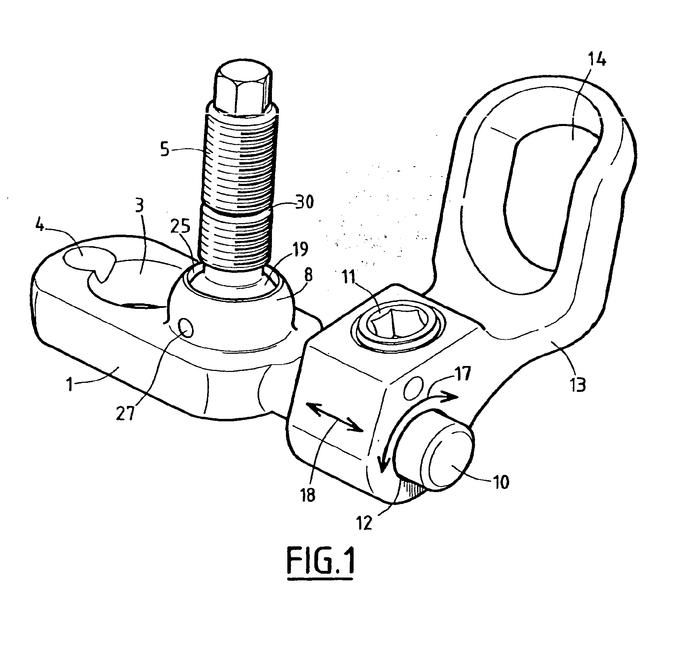

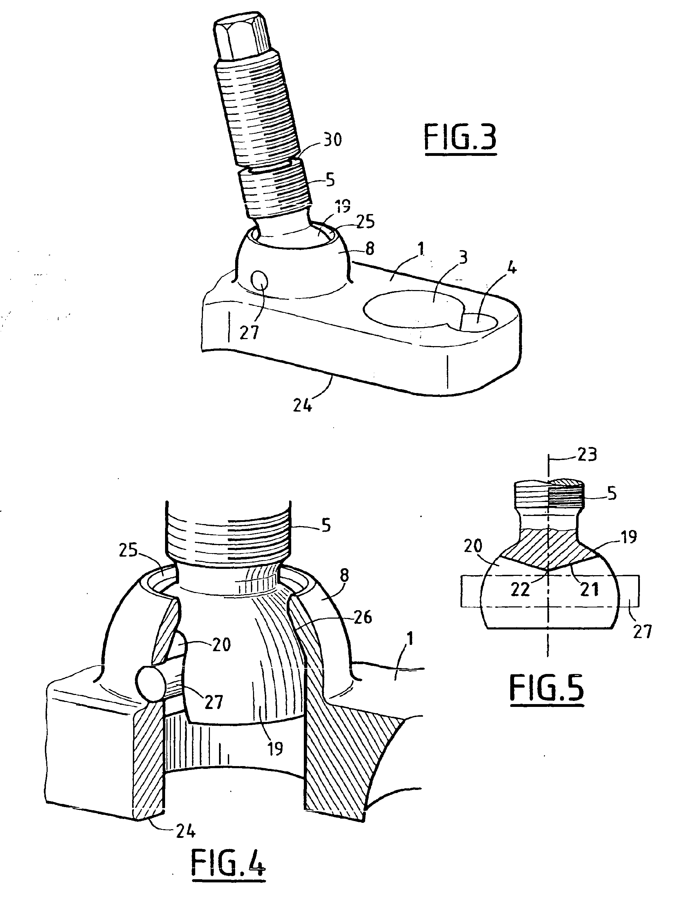

The first illustrative embodiment of the invention shown in Figures 1 to 7 is an implant made up of two parts.

The first part is a sacral plate 1 intended to be fixed on the sacrum of a patient, typically on vertebra S1. It is fixed by means of a bone anchoring screw 2 which passes through an aperture 3 in the sacral plate 1. In the example shown, the plate 1 also comprises an internally threaded hole 4 in which a threaded plug (not shown) can be screwed in order to immobilize the head of the screw 2 and prevent it from migrating from its seat after placement. The sacral plate 1 also comprises a threaded protrusion 5 on which a slotted connector 6 is customarily intended to be fitted, which slotted connector 6 is itself traversed by the rod 7 of the device for correcting deformations of the spine, of which the invention forms an integral part. This protrusion can be fixed and substantially perpendicular to the upper surface of the sacral plate 1 or, as in the example shown, it can be of the type referred to as "multi-axial", that is to say capable of being oriented inside a cone whose angle is normally of the order of 30°. The means ensuring this multi-axial orientation can be of various known types, but the preferred example shown, which is novel, will be described in more detail below. The connector 6 is immobilized on the dome 8, carried by the sacral plate 1, by means of a nut 9, which also allows the slotted connector 6 to clamp the rod 7.

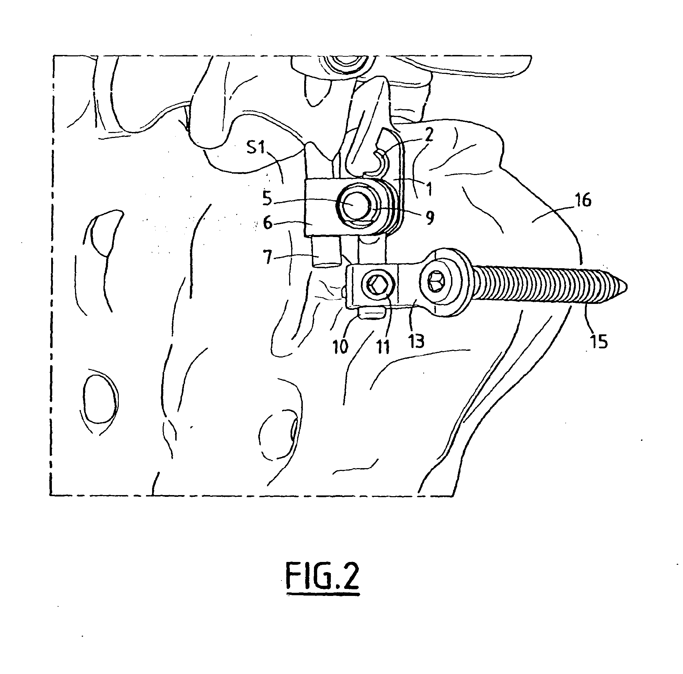

The sacral plate 1 also comprises a longitudinal rod 10 which continues it at its end intended to be directed towards the lower part of the sacrum. This longitudinal rod 10 is intended to be inserted into a longitudinal orifice 12 of a lateral extension plate 13 and to be immobilized therein by a threaded plug 11. This lateral extension plate 13 is designed so that its aperture 14 permits implantation of a bone anchoring screw 15 which penetrates into an iliac bone 16 of the patient in such a way as to fix the lateral extension plate 13 there (the screw 15 can be seen in Figure 2).

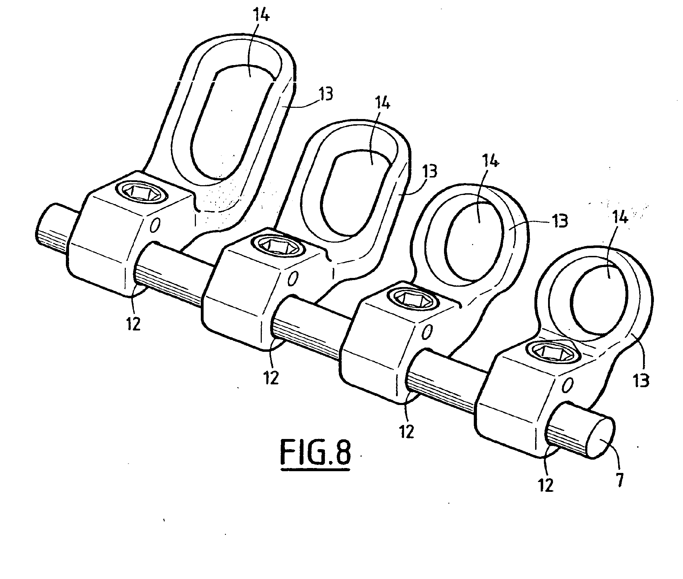

Compared to known sacral implants in one piece, the implant in two parts according to the invention has several advantages. Its geometry can be varied. It can be modified by virtue of the choice of position of the lateral extension plate 13 on the longitudinal rod 10 of the sacral plate 1, both in rotation and in translation, as indicated by the arrows 17, 18 in Figure 1. Moreover, it is also possible to choose the lateral extension plate 13 from several models with different geometries and sizes, such as those shown in Figure 8, which are fitted on the same rod 7. The geometry of the implant can thus be optimally adapted to the particular morphology of the patient and to the exact site of fixation of the implant. Moreover, it is possible to achieve a whole variety of implant geometries with the aid of a single model of sacral plate 1. This simplifies organization of the production and management of stocks of component parts.

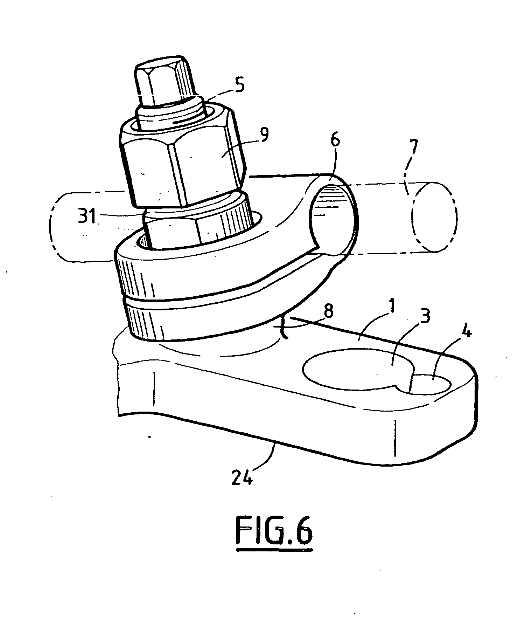

In the preferred example shown, the lower part of the protrusion 5 comprises a spherical surface 19 and is traversed by a slot 20 substantially perpendicular to the protrusion 5. As will be seen from Figure 5, this slot 20 is delimited by an upper surface 21 of V shape whose point 22 is situated substantially on the axis of symmetry 23 of the protrusion 5.

To assemble the protrusion 5 and the plate 1, the protrusion 5 is engaged through the lower face 24 of the plate 1 and is passed through an orifice 25 arranged at the summit of the dome 8. The spherical surface 19 of the lower part of the protrusion 5 thus comes into contact with the inner surface 26 of the dome 8. A pin 27 is then fitted which passes through the dome 8 and comes to lie in the slot 20 traversing the lower part of the protrusion 5. As will be seen from Figure 5, the point 22 of the V constituting the upper surface delimiting the slot 20 must lie just above the pin 27, so as to permit only very slight vertical clearance of the protrusion 5 inside the dome 8. The pin 27 has a diameter practically equal to the width of the slot 20 so as to prevent any significant rotation of the protrusion 5 about its axis 23.

Another particular feature of the invention is that the inner surface 26 of the dome 8 is not spherical, but conical. This is because such a shape, combined with the spherical shape of the surface 19 of the lower part of the protrusion 5, makes it possible to optimize the wedging of the protrusion 5 in the dome 8 after the nut 9 has been tightened. With this cone-to-sphere contact, the pressure due to the clamping is distributed over a smaller surface area than if there were a sphere-to-sphere contact. The clamping is thus more effective, and the possibility of one surface sliding on the other is further reduced. The rigidity of the assembly is thus improved.

These configurations of the lower part of the protrusion 5 and of the inner surface of the dome 8 provide for a complete multi-axial orientation of the protrusion 5 (typically inside a cone with an angle of about 30°) and excellent immobilization of this protrusion 5.

Depending on the configuration given to the connector 6, it will be possible, after tightening the nut 9:
- either to return the protrusion 5 automatically to an orientation substantially perpendicular to the plate 1; the multi-axial nature of the protrusion 5 will thus have served only during implantation of the corrective device;
- or to retain the protrusion 5 in an orientation not perpendicular to the plate 1 even after the nut 9 has been tightened (case shown in Figure 6).

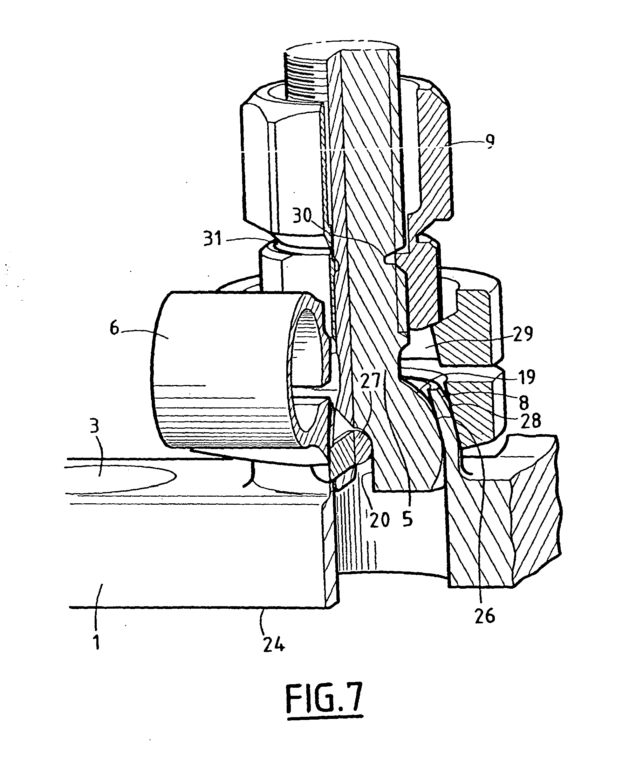

For reasons analogous to the above, it is also advantageous that the contact between the spherical outer surface of the dome 8 and the connector 6 is a sphere-to-cone contact. To this end, as is shown in Figure 7, the surface 28 delimiting the orifice 29 of the connector 6 traversed by the protrusion 5 can be given a conical shape. In this way, the assembly is assured maximum rigidity.

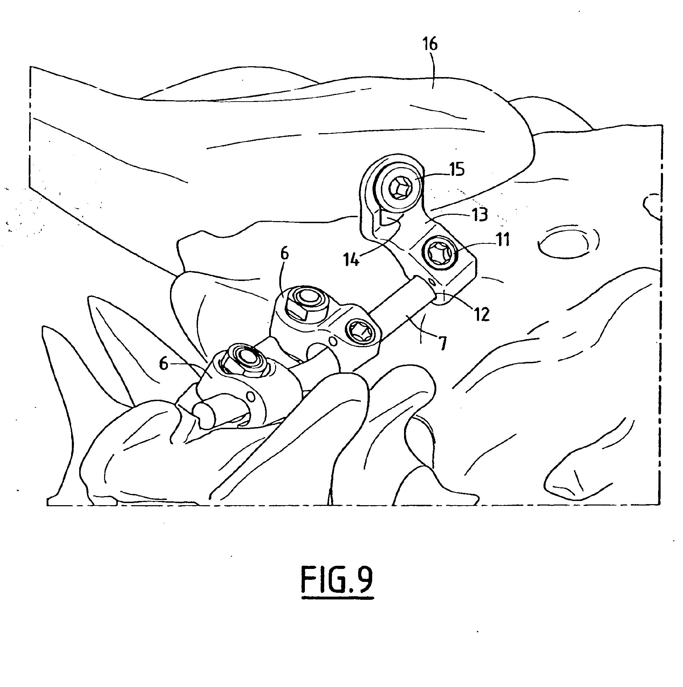

An implant not made according to the invention is shown in Figure 9. It consists in using, as implant fixed on an iliac bone 16 by a screw 15, a lateral extension plate 13 of the above type, in whose orifice 12 the rod 7 of the osteosynthesis device is directly inserted. In the example shown, the latter is fixed to other parts of the spine by slotted connectors 6, customarily mounted on protrusions carried by bone anchoring screws.

Various modifications of the invention are conceivable. In particular, it is not essential for the orifice 12 of the lateral extension plate to encircle the rod 7, 10 over its entire circumference, as is illustrated. It suffices for the plug 11 or any other equivalent means to wedge the rod 7, 10 against the wall of the orifice 12. Likewise, for fixing the different parts of the implant on the spine or on an iliac bone, it would be possible to use means other than bone anchoring screws.

After the osteosynthesis device comprising the implants according to the invention has been fitted in place, the surgeon generally removes those parts of the protrusion 5 and of the nut 9 which are redundant. To do so, lines of lesser resistance in the form of notches 30, 31 are preferably formed on these elements, as is known. It is also possible to arrange such lines of lesser resistance on the longitudinal rod 10 of the sacral plate 1 so that, if necessary, it is easy to shorten the part of the rod 10 extending beyond the lateral extension plate 13. However, care must be taken to ensure that these lines of lesser resistance do not significantly impair the mechanical strength of the rod 10 during the stresses to which it is subjected after the device has been fitted.

## Claims

1. Implant for a spinal osteosynthesis device, comprising a first part having a longitudinal orifice (12) and means for immobilizing a rod (7, 10) inside said longitudinal orifice (12), and a lateral extension (13) provided with an aperture (14) for the passage of a bone anchoring screw (15), and designed so that said screw (15) can fix it on an iliac bone ot the patient, **characterized in that** it comprises a second part formed by a sacral plate (1) provided with means (3) for fixing it on the sacrum of the patient, with a longitudinal rod (10) which can be fitted into and immobilized in rotation and in translation in the longitudinal orifice (12) of said first part, and with a threaded protrusion (5) for fitting of a connector (6).

2. Implant according to Claim 1, **characterized in that** said immobilizing means are formed by an internally threaded hole and a threaded plug (11) screwed into said internally threaded hole.

3. Implant according to Claim 1 or 2, **characterized in that** said protrusion can have a multi-axial orientation inside a cone.

4. Implant according to Claim 3, **characterized in that:**
- said implant comprises a dome (8) whose inner surface (26) is of conical shape;
- the surface (19) of the lower part of the protrusion (5) , intended to be held inside said dome (8) and to come into contact with said inner surface (26) of the dome (8), is of spherical shape and is traversed by a slot (20) delimited by an upper surface (21) of V shape with the point (22) directed towards the lower end of the protrusion (5); and
- said dome (8) and said slot (20) are traversed by a pin (27).

5. Implant according to Claim 4, **characterized in that** the dome (8) has a spherical outer surface.

6. Assembly formed by an implant for a spinal osteosynthesis device, a slotted connector (6) for attachment to a rod (7), of the type comprising an orifice (29) for fitting said connector (6) on said protrusion (5), and a nut (9) for immobilizing said connector (6) on said implant and for clamping said rod (7) in said connector (6), and in that said implant is of the type according to one of Claims 1 to 5.

7. Assembly according to Claim 6, **characterized in that** the implant is of the type according to Claim 5, and **in that** the surface (28) delimiting said orifice (29) of the connector (6) is of conical shape.

## Patentansprüche

1. Implantat für eine Wirbelsäulen-Osteosynthese-Vorrichtung, welches aufweist: ein erstes Teil, welches eine Längs-Öffnung (12) und Mittel zum Immobilisieren einer Stange (7, 10) in der Längs-Öffnung (12) hat, und einen seitlichen Fortsatz (13), welcher mit einem Durchlass (14) für die Passage einer Knochen-Ankerschraube (15) versehen ist und so ausgebildet ist, dass derselbe mit der Schraube (15) an einem Darmbein des Patienten befestigbar ist, **dadurch gekennzeichnet, dass** es ein zweites Teil aufweist, welches von einer Kreuzbein-Platte (1) ausgebildet ist, welche mit Mitteln (3) zum Befestigen am Kreuzbein des Patienten, mit einem Längs-Stab (10), welcher in der Längs-Öffnung (12) des ersten Teils einpassbar und gegen Drehen und Translation immobilisierbar ist, und mit einem Gewinde-Vorsprung (5) zum Aufschrauben eines Anschlussstücks (6) versehen ist.

2. Implantat gemäß Anspruch 1, **dadurch gekennzeichnet, dass** die Immobilisierungs-Mittel von einem Innengewinde-Loch und einem Gewinde-Bolzen (11) ausgebildet sind, welcher in das Innengewinde-Loch eingeschraubt wird.

3. Implantat gemäß Anspruch 1 oder 2, **dadurch gekennzeichnet, dass** der Vorsprung eine mehrachsige Ausrichtung in einem Konus haben kann.

4. Implantat gemäß Anspruch 3, **dadurch gekennzeichnet, dass**:
- das Implantat eine Kuppel (8) aufweist, deren Innenfläche (26) konisch ist,
- die Fläche (19) des unteren Teils des Vorsprungs (5), welche zum Halten in der Kuppel (8) und zum In-Kontakt-Kommen mit der Innenfläche (26) der Kuppel (8) vorgesehen ist, kugelförmig ist und von einem Schlitz (20) durchquert ist, welcher von einer oberen Fläche (21) begrenzt, welche V-förmig ist, wobei die Spitze (22) hin zum unteren Ende des Vorsprungs (5) gerichtet ist, und
- die Kuppel (8) und der Schlitz (20) von einem Stift (27) durchquert sind.

5. Implantat gemäß Anspruch 4, **dadurch gekennzeichnet, dass** die Kuppel (8) eine kugelförmige Außenfläche hat.

6. Anordnung, welche von einem Implantat für eine Wirbelsäulen-Osteosynthese-Vorrichtung, von einem geschlitzten Anschlussstück (6) zum Anbringen an einem Stab (7), wobei das Anschlussstück (6) von dem Typ mit einer Öffnung (29) zum Anbringen desselben an dem Vorsprung (5) ist, und von einer Mutter (9) zum Immobilisieren des Anschlussstücks (6) an dem Implantat und zum Festklemmen des Stabes (7) in dem Anschlussstück (6) ausgebildet wird, und wobei das Implantat von dem Typ gemäß einem der Ansprüche 1 bis 5 ist.

7. Anordnung gemäß Anspruch 6, **dadurch gekennzeichnet, dass** das Implantat von dem Typ gemäß Anspruch 5 ist und das die Fläche (28), welche die Öffnung (29) des Anschlussstückes (6) beschränkt, konisch ist.

## Revendications

1. Implant pour un dispositif d'ostéosynthèse rachidienne comprenant une première partie ayant un orifice longitudinal (12) et des moyens pour immobiliser une tige (7, 10) à l'intérieur dudit orifice longitudinal (12), et une extension latérale (13) munie d'une ouverture (14) pour le passage d'une vis d'ancrage osseux (15), et conçue de sorte que ladite vis (15) puisse se fixer sur un os iliaque du patient, une deuxième partie formée d'une plaque sacrée (1) munie de moyens (3) pour la fixer sur le sacrum du patient, avec une tige longitudinale (10) qui peut être ajustée et immobilisée en rotation et en translation dans l'orifice longitudinal (12) de ladite première partie, et avec une saillie filetée (5) pour l'ajustement d'un connecteur (6).

2. Implant selon la revendication 1, **caractérisé en ce que** lesdits moyens pour immobiliser sont formés par un trou fileté de manière interne et un bouchon fileté (11) vissé dans ledit trou fileté de manière interne.

3. Implant selon la revendication 1 ou 2, **caractérisé en ce que** ladite saillie peut avoir une orientation multi axiale à l'intérieur d'un cône.

4. Implant selon la revendication 3, **caractérisé en ce que** :
ledit implant comprend un dôme (8) dont la surface intérieure (26) est de forme conique;
la surface (19) de la partie inférieure de la saillie (5), destinée à être maintenue à l'intérieur dudit dôme (8) et à venir en contact avec ladite surface intérieure (26) du dôme (8), est de forme sphérique et est traversée par une fente (20) délimitée par une surface supérieure (21) en forme de V avec la pointe (22) dirigée vers l'extrémité inférieure de la saillie (5); et
ledit dôme (8) et ladite fente (20) sont traversés par une goupille (27).

5. Implant selon la revendication 4, **caractérisé en ce que** le dôme (8) a une surface extérieure sphérique.

6. Ensemble formé par un implant pour un dispositif d'ostéosynthèse rachidienne, un raccord fendu (6) pour la fixation à une tige (7), du type comprenant un orifice (29) pour ajuster ledit raccord (6) sur ladite saillie (5), et un écrou (9) pour immobiliser ledit raccord (6) sur ledit implant et pour serrer ladite tige (7) dans ledit raccord (6), et en ce que ledit implant est du type selon l'une quelconque des revendications 1 à 5.

7. Ensemble selon la revendication 6, **caractérisé en ce que** l'implant est du type selon la revendication 5, et **en ce que** la surface (28) délimitant ledit orifice (29) du raccord (6) est de forme conique.
